# EUROPEAN PATENT APPLICATION

(11) **EP 3 767 296 A1**
(43) Date of publication of application: **20.01.2021**
(21) Application number: 19186494.1
(22) Date of filing: 16.07.2019
(51) Int. Cl.: G01N 33/574, G01N 33/50

(54) **3D-1D MULTIDIMENSIONAL CANCER-ON-A-CHIP**

(71) Applicant: Friedrich-Alexander-Universität Erlangen-Nürnberg, 91054 Erlangen (DE)
(72) Inventor: LEAL-EGANA, Aldo, 90443 Nürnberg (DE); BOCCACCINI, Aldo Roberto, 91054 Erlangen (DE)
(74) Representative: Vossius & Partner Patentanwälte Rechtsanwälte mbB

(57) **Abstract**

The present invention relates to an apparatus for analyzing single tumor cells, said apparatus comprising a hydrogel-based polymeric microcapsule for entrapping cells and a hydrogel having on its surface a single cell migration path being formed by one or more extracellular matrix proteins and/or one or more polymers comprising RGD peptides or proteins, wherein the polymeric microcapsule and the path are placed in the apparatus such that upon the release of cells from the polymeric microcapsule a single tumor cell can migrate from the polymeric microcapsule on the path.

## Description

The present invention relates to an apparatus for analyzing single tumor cells, said apparatus comprising a hydrogel-based polymeric microcapsule for entrapping cells and a hydrogel having on its surface a single cell migration path being formed by one or more extracellular matrix proteins and/or one or more polymers comprising RGD peptides or proteins, wherein the polymeric microcapsule and the path are placed in the apparatus such that upon the release of cells from the polymeric microcapsule a single tumor cell can migrate from the polymeric microcapsule on the path. The present invention also relates to a kit for analyzing single tumor cells comprising (i) a hydrogel-based polymeric microcapsule for entrapping cells, (ii) a hydrogel having on its surface a single cell migration path being formed by one or more extracellular matrix proteins and/or one or more polymers comprising RGD peptides or proteins, and (iii) optionally instructions how to use the kit.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Cancer is a leading cause of morbidity and mortality worldwide, with approximately 14 million new cases and 8.2 million cancer-related deaths reported in 2012 (World Health Organization).

Most type of cancers *(i.e.* breast, prostate, brain, bone, etc.) are characterized by the presence of three-dimensional semisolid structures (3D) composed by extracellular matrix proteins, known as tumors (1, 2). Tumors could restrict cell proliferation and migration of malignant cells (2, 3). However, through a combination of biomechanical forces *(i.e.* traction and indentation forces) and the secretion of matrix metalloproteinases, these cancerous cells can survive and are released from the tumor in a process named intravasation (1-6). During intravasation cells can migrate into blood and lymph vessels with the purpose to invade other organs (known as metastasis) (4, 5). It is important to note that cell migration from tumors is directed by the existence of aligned fibrils made of extracellular matrix proteins (having one-dimensional morphology, named as 1D), guiding cancerous cells to be released from tumors and to generate metastasis (7, 8).

Currently, one of the greatest complications to fight cancer is the existence of tumor heterogeneity. Tumor heterogeneity can be described as a hierarchical organization of cancer cells within a tumor, where sub-populations of cells with the same genotype, but a different phenotype, coexist within the same niche (9, 10). Tumor heterogeneity can be phenotypically detected by differences in cell morphology, proliferation and migration rate, pharmacologic sensitivity and the expression of surface markers, to name a few examples (9, 14). These characteristics can be used as cell signatures to distinguish between different sub-populations localized within the same niche.

Since the tumor microarchitecture is constantly remodeled *in vivo,* it is extremely difficult to study tumor heterogeneity *in situ.*

At present, techniques such as whole-genome sequencing of the entire cell population, (11, 12) and proteomic characterization of micro-biopsies (12, 15) are the two most reliable *ex-situ* methods available to study tumor heterogeneity. These assays of tumor heterogeneity are based on the detection of single clones and/or subpopulations within an entire population.

In addition, many current *in vitro* strategies to study the phenomenon of tumor heterogeneity result in failures. For example, Western blot or qPCR average the cell behavior across the entire population masking responses specific to a certain sub-type.

Thus, single-cell assays have gained attention, due to the possibility to test the expression of several phenotypic characteristics by using motorized microscopes *(i.e.* confocal microscope), developing a new generation of High-Throughput-Screening Test, in micro-platforms traditionally named *Lab-on-a-Chip* (17-19). However, at present there are no *in vitro* alternatives to study the behavior of single cell migrating from complex 3D cultures to 1D guidelines (i.e. multicellular spheroids, immobilized cocultures, heterogeneous populations found in a 3D scaffold and further migrating as single cells), due to the difficulty to fragment multicellular populations and to study single cells sourced from these 3D cultures, without losing their mechanical and biophysical stimulation originally sensed by entrapped cells in the three-dimensional milieu, as well as keeping the cells alive and without further damage.

Moreover, there is a lack of soft systems (*i.e. in vitro* polymer-based platforms having elasticities below to MPa or GPa, measured as Young's moduli) allowing the separation of multicellular populations, and further *in vitro* culture of such cells under mechanical properties mimicking the neoplastic niche which is characterized for having values of elasticity up to 150 kPa, measured as Young's moduli (20). In this regard it is important to note the relevance of the mechanical stimulation on cancer cells, in order to properly mimic the environmental stimulation observed *in vivo* (2, 4, 5).

Noteworthy, even though it is possible to find some hydrogel-based scaffolds mimicking the soft environment surrounding cancer cells (21), most *in vitro* systems used nowadays consist on two-dimensional flat surfaces (20) with elasticities in the range of MPa to GPa (*i.e*. Glass, cell culture plates) (22), 2D flat hydrogels having values of elasticity below to 20 kPa (Du Chez, B. J., et al. (2019). Durotaxis by Human Cancer Cells. Biophysical Journal. 116, 670-683), or 3D biodegradable hydrogels having elasticity below to 1.0 kPa (*i.e.* Matrigel; reference: https://www.corning.com/media/worldwide/cls/documents/applications/CLS-AC-AN-449%20DL.pdf). which mismatch the stiffness found in the tumoral environment, and therefore, most of current scaffolds used to mimic the neoplastic architecture lack of the mechanical stress induced by the confinement, which is involved in the stimulation of their pathogenic potential, including the expression of tumor heterogeneity. Furthermore, if some of these scaffolds can mimic the mechanical properties of the neoplastic niche, these fail when resembling the topographic characteristics of the malignant milieu (23, 24) which however, are required for properly characterizing the pathogenicity of cancer cells *in vitro.*

Therefore, an apparatus and a kit are needed that can properly mimic the mechanical and topographical stimulation found *in vivo,* allowing the expression of tumor heterogeneity *in vitro,* with the final purpose to use this device and establish the described protocols as a new type of clinical test to study pathogenicity of cancer cells *in vitro* or *ex-situ.*

Due to the complexity to analyze cell migration *in vivo,* there is also need for systems enabling studying the displacement of cells from a three-dimensional architecture to a linear system (i.e. such as observed during intravasation of cancer cells) *in vitro.* These needs are addressed by the present invention.

Accordingly, the present invention relates in a first aspect to an apparatus for analyzing single tumor cells, said apparatus comprising a hydrogel-based polymeric microcapsule for entrapping cells and a hydrogel having on its surface a single cell migration path being formed by one or more extracellular matrix proteins and/or one or more polymers comprising RGD peptides or proteins, wherein the polymeric microcapsule and the path are placed in the apparatus such that upon the release of cells from the polymeric microcapsule a single tumor cell can migrate from the polymeric microcapsule on the path.

Tumor cells (or neoplastic cells) are abnormal cells that can divide essentially relentlessly and form either solid or non-solid tumors *in vivo.* Tumor cells keep dividing and growing over time, under cell culture conditions in a laboratory and in particular when cultured in a microcapsule as described herein. Tumor cells can either grow benign (generally harmless) or cancerous. In the latter case, tumor cells are cancerous, i.e. cancer cells. The tumor cells are preferably cancer cells. The cancer cells may be metastatsic cancer cells. Accordingly, tumor cells are also preferably malignant cells.

The tumor cells may be cultured tumor cells or tumor cells that were directly obtained from an *in vivo* tumor. In the latter case the tumor cells are generally comprised in a tumor sample (i.e. a biopsy from tumor tissue). In tumor samples there are two different types of cells, tumor cells (or neoplastic cells) and cells being associated with the tumor cells (i.e. in tumor tissue). With respect to the cells being associated with the tumor cells, these are also cells found within tumors, but they do not have the capability to produce a neoplastic disease. It has been described that cells being associated with the tumor cells support the progression of the neoplastic disease. Examples of cells being associated with the tumor cells are cancer associated fibroblasts and endothelial cells. In case the tumor cells are comprised within a tumor sample it is, thus, to be understood that the apparatus of the invention offers the possibility to study both the tumor cells as well as the cells being associated with the tumor cells.

The term "hydrogel" as used herein refers to a two- or multi-component system consisting of a three-dimensional network of polymer chains and water that fills the space between macromolecules. Depending on the properties of the polymer (or polymers) used, as well as on the nature and density of the network joints, such structures in an equilibrium can contain various amounts of water; typically in the swollen state. The mass fraction of water in a hydrogel is much higher than the mass fraction of a polymer. In practice, to achieve high degrees of swelling, it is common to use synthetic polymers that are water-soluble when in non-cross-linked form. Hydrogels may be synthesized in a number of chemical ways. These include one-step procedures like polymerization and parallel cross-linking of multifunctional monomers, as well as multiple step procedures involving synthesis of polymer molecules having reactive groups and their subsequent cross-linking, possibly also by reacting polymers with suitable cross-linking agents. A polymer engineer can design and synthesize polymer networks with molecular-scale control over structure such as cross-linking density and with tailored properties, such as biodegradation, mechanical strength, and chemical and biological responses to stimuli. A review of the preparation, characterization, and applications of hydrogels can be found, for example, in Ahmed et al. (2015), Journal of Advanced Research, 6(2):105-121. The hydrogel having on its surface a single cell migration path is preferably a flat hydrogel. A "flat hydrogel" as referred to herein is a hydrogel having a planar surface. The hydrogel is also preferably made of non-cell adhesive polymers, so that the polymers of the hydrogel cannot interfere with the migration of the cells along the path.

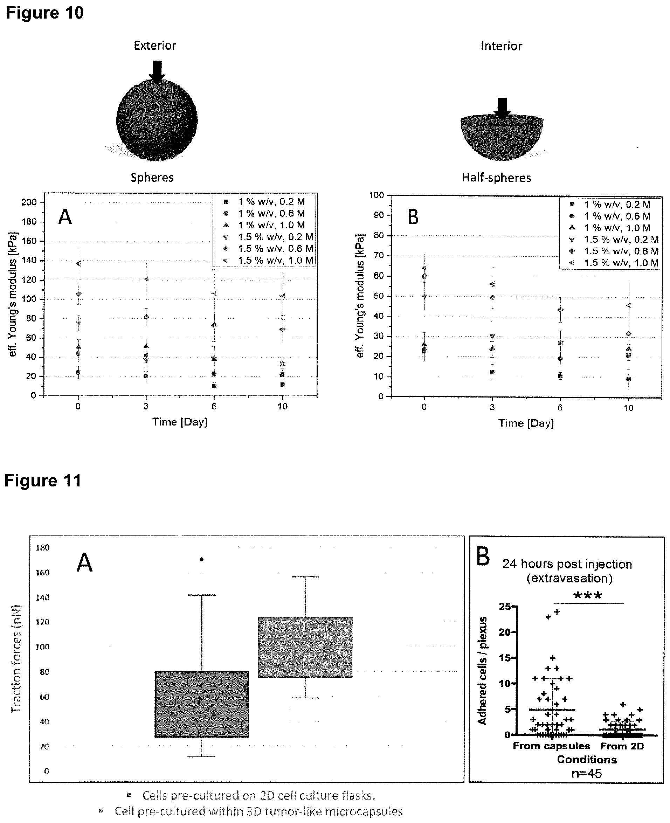

A "hydrogel-based polymeric microcapsule for entrapping cells" as referred to herein is a three-dimensional (3D) spherical structure made up at least in part by polymers that build a hydrogel and being capable of entrapping tumor cells. The hydrogel-based polymeric microcapsule for entrapping cells exhibit mechanical properties (i.e. elasticity, measured as Young's moduli) mimicking *in vivo* tumors and may therefore also be referred to as "tumor like microcapsules". Determination of elastic properties of tumor like microcapsules preferably shows values between 10 to 140 kPa in the exterior, and from 9 to 70 kPa in the interior, resembling the values reported for several tumoral tissues (Malgorzata Lekka, 2016, BioNanoScience, 6: 65-80). Figure 10 shows some exemplary measurements of elasticity of the tumor-like microcapsules.

The tumor cells can be released from the capsule when the capsule is, for example, degraded, broken up, disrupted, melted, or dissolved. In this connection "degraded" means any impairment in respect to at least one physical or chemical property of the microcapsule, so that cells entrapped in the microcapsule are released from the microcapsule. The capsule can be generally broken up or disrupted by biological or biomechanical forces which are generated by the cells growing in the capsule. For instance, the cells may reach a cell density within the capsule so that forces break up or disrupt the capsule. It is therefore preferred that the cells are released from the microcapsule either upon degradation of the microcapsule or upon the breakup or disruption of the microcapsule by the described forces. The aspect of degradation will be further detailed herein below.

It is also possible that the microcapsule has pores for releasing the cells. The cells can transmigrate through the pores. The pore size is generally selected such that the cells can only pass the pores once the cells were grown in the capsule, for example, such that biological or biomechanical forces are generated which press the cells through the pores. For instance, the forces may mediate cell movement or cell displacement arranging the cytoskeleton of the cell or modifying the cell morphology, so that cells can pass the pores.

The purpose "for entrapping cells" defines that the microcapsule has dimensions which are suitable to incorporate one or more cell types (i.e. different population of cells) and preferably also for growing the cell(s) within the degradable polymeric microcapsule.

A "single cell migration path" as referred to herein is a one-dimensional (1D) structure (i.e. a surface) on the surface of the hydrogel being formed by one or more extracellular matrix proteins and/or one or more polymers comprising RGD (Arginylglycylaspartic acid) peptides or proteins. The designation "single cell" defines that the patch has dimensions only allowing a single cell released from the microcapsule to attach to, spread on or migrate along the path. The width of the path is not sufficiently broad so that two cells from the microcapsule can migrate in parallel along the path nor so small that not a single cell from the microcapsule fits onto the patch. Of course, more than one cell can migrate one after another along the patch. Moreover, the length of the patch is sufficiently long, so that each single cell can be analyzed separately long the path. Single cell migration path can be prepared for example, by soft micropatterning (25).

The kind of the single tumor cell analysis for which the apparatus of the invention is suitable is not particularly limited and depends on the experimental needs of the user of the apparatus of the invention. Non-limiting examples are the analysis of genomics, transcriptomics, proteomics and metabolomics at the single cell level or considering all single cells as a whole population, or having several sub-populations. Further examples will be described herein below.

Extracellular matrix (ECM) is the non-cellular component that provides structural scaffolding to surrounding cells. The ECM is composed *inter alia* of water, fibrous proteins and polysaccharides. The main fibrous ECM proteins are collagens, elastins, fibronectins and laminins. Collagen is the main structural protein in the extracellular space and is the most abundant protein in the ECM. Collagens are divided into two classes - fibril (types I, II, III, V) and non-fibril (types IV, VI). Elastin is a highly elastic protein in connective tissue and allows many tissues in the body to resume their shape after stretching or contracting. Fibronectin is a high molecular weight ECM glycoprotein composed of two nearly identical monomers (220-250 kDa) linked by disulfide bonds. It is present in two forms - the soluble form (the major component of blood plasma which is mainly produced by hepatocytes) and insoluble form (a major component of the ECM which is secreted by various cells prior to being assembled into an insoluble matrix). By binding to other ECM components such as collagen, integrins, and heparin, fibronectin plays an important role in cell adhesion, migration, growth, and differentiation. Laminins are high-molecular weight ECM proteins that are a major component of the basal lamina of the basement membrane. They consist of an α-chain, a β-chain, and a γ-chain which form a cross-like structure that bind to cell membranes and other ECM proteins. Laminins contribute to cell attachment, differentiation, and motility.

Arginylglycylaspartic acid (RGD) is the principal integrin-binding domain present within ECM proteins, such as fibronectin, vitronectin, fibrinogen, osteopontin, and bone sialoprotein (Bellis, Biomaterials 2011; 32(18):4205-4210). RGD is also present in some laminins and collagens. The RGD sequence can bind to multiple integrin species, and synthetic RGD peptides and proteins are advantageous for several biomaterials applications including the application as a component of a single cell migration path as described herein. RGD is the most widely studied adhesive peptide in the biomaterials field. Synthetic RGD peptides and proteins offer the advantage of having the ability to tightly control the density, patterning, structure and orientation of the patch and also provide rich opportunities for inducing cell responses along the patch that would not normally be evoked by native ECMs.

The requirement that the hydrogel has on its surface a single cell migration path means that the single cell migration path is joined, fastened or connected to the hydrogel.

The requirement that the microcapsule and the path are placed in the apparatus such that upon the release of cells from the polymeric microcapsule a single tumor cell can migrate from the polymeric microcapsule on the path requires that a cell released from the microcapsule within the apparatus can reach the path. As will be discussed in more detail herein below the cells may be released from the microcapsule as single cells or as spheroids. In the latter case the spheroids release or are transformed into single cells which then attach to the patch. This can be achieved, for example, by co-localizing the microcapsule and the path on the hydrogel, by positioning or placing the microcapsule and the path in direct contact with or without linking the microcapsule and the path or by connecting the microcapsule and the path. Connecting the microcapsule and the path is preferred and means that the microcapsule and one end of the path co-localize and are connected to each other, usually via chemical bonds. The connection has dimensions which allow a single cell to migrate from the polymeric microcapsule on the path once cells are released from the polymeric microcapsule.

It is to be noted that the apparatus of the invention may not only harbor one microcapsule and only one migration path but preferably several microcapsules and several migration paths. Similarly, it is preferred that that not only one migration path is co-localized with, positioned or placed in direct contact with or connected to (each) microcapsule but several migration paths. Accordingly, it is increasingly preferred that the apparatus comprises at least 2, at least 3, at least 4, at least 5, at least 10, at least 20, at least 50 or at least 100 microcapsules. It is also increasingly preferred that each microcapsule is co-localized with, positioned or placed in direct contact with or connected to at least 2, at least 3, at least 4, at least 5, at least 10, at least 20, at least 50 or at least 100 migration paths.

Since the migration paths are typically prepared separately from the microcapsules, the apparatus of the invention can be individually designed and performed, having specific properties/characteristics according to the operator's wishes. Also different elasticities, pore size distribution and/or composition can be selected. Likewise, the pattern of the migration paths and microcapsules with different motives and geometries (i.e.. conical or sigmoidal migration lines) can be adjusted to the respective experimental needs. Finally, the apparatus offers the possibility that the operator can choose the localization of the microcapsules and paths within the system and, for example, on the hydrogel which allows generating automatized analytic protocols.

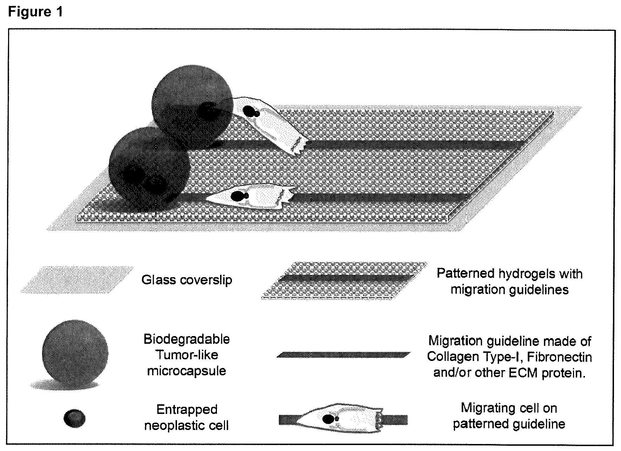

As can be taken from the examples herein below, it was surprisingly found that a novel type of miniaturized platform (so called 3D-1D Multidimensional Cancer-on-a-Chip; Figure 1) constituted by the above described microcapsule and hydrogel having on its surface a migration path is ideal to study heterogeneity among a complex population of tumor cells.

It is important to note that the biophysical properties within the microcapsule described herein only allow tumor cells to grow and to be released from the microcapsule. Surprisingly, it was found that healthy and/or non-tumor cells are not able to proliferate within the microcapsule, are not capable to degrade the polymer forming the microcapsules or do not produce enough forces to transmigrate from, disrupt or break-up the microcapsules. Healthy and/or non-tumor cells that do not proliferate die over time within the microcapsule due to the lack of oxygen and/or the high mechanical stress as a result of the entrapment. Hence, it was surprisingly found that the microcapsule is "selective" for the release of tumor cells.

Within the microcapsule tumor cells or a mixture of tumor and healthy cells can be entrapped whereas only tumor cells are released from the microcapsule. As will be further evident from the method of the invention as described herein below, the cells are entrapped in the microcapsule when using the apparatus of the invention for analyzing single tumor cells. The cells can be entrapped, for example, by injecting the cells into the microcapsule. As a commercial product the apparatus of course does not yet comprise cells being entrapped in the microcapsule. Hence, a complex and multicellular cell population (i.e. a biopsy), a single cell type (monoculture) as well as multiple cell types (co-culture) can be entrapped. Within the microcapsule tumor cells can be cultured under mechanical properties which closely resemble those found in *in vivo* tumor tissues. In particular, the oxygen tension and/or distribution as well as the mechanical forces (i.e. stiffness, solid stress or cell confinement) within *in vivo* tumor tissue can be very well mimicked. The migration paths provide thin migration guidelines on the top of the hydrogel. Migration guidelines have a width that only allows single-cell migration. Hence, in the practical use of the apparatus of the invention once cells have been entrapped in the microcapsule and covered by cell culture media, the apparatus is incubated under cell culture conditions for an experimental time decided by the researcher/operator. During the incubation time, the tumor cells grow and generally also generate multicellular spheroids within the microcapsule. Such cells and spheroids are characterized by a biological behavior resembling those found in *in vivo* tumors, including having cellular heterogeneity which closely resembles the cellular heterogeneity of tumor cells in *in vivo* tissue, in particular with respect to mechanical stimulation and the confinement or lack of oxygen (28-30). It is noted that culturing the cells in 3D in the microcapsule significantly more closely resembles the tumor cells found in *in vivo* tissue as compared to culturing them "traditionally" on 2D surfaces (i.e. a cell culture flask).

For example, after 5 days of culture (actually depending on the cell type), tumor cells are released from the capsules. The start of migration can be observed as single tumor cells or cellular microspheroids of tumor cells which are localized at the capsule-edge. After release, and due to gravity, single tumor cells reach the migration path. During this stage, single cells reach the migration path, anchor to them, spread or start migrating. When cellular aggregates released from the capsules reach the migration path they are generally fragmented into single tumor cells, which attach and spread on the migration path or the migration paths in case several of them are co-localized with, positioned or placed in direct contact with or connected to one microcapsule. The migration path is formed by extracellular matrix proteins and/or RGD proteins or peptides, so that the cells tumor migrate down the path (for review: Rauch and Schäfer, Eur J Pediatr Surg. 2003 Jun;13(3):158-62.). On the migration paths, for example, cellular attachment, spread, and further migration of single tumor cells can be studied. Cells forming multicellular aggregates that cannot fragment or single tumor cells not reaching the paths are not able to attach to the paths, and therefore, may be removed, for example, during a media change. Non-adherent cells may also die due to the lack of cell adhesion.

Through the stages of adhesion, spreading and migration along the path single tumor cells can be advantageously characterized. Several characterization strategies to determine tumor heterogeneity after analyzing single cells can be named, for example, by biophysical, toxicological and/or biochemical methods (i.e. determining the exertion of traction forces, indentation forces, migration speed, cell morphology, sensitivity to chemotherapeutic drugs, sensitivity to protease inhibitors, expression of surface markers, etc.). Because single tumor cells are constantly delivered from the microcapsule, experimental assays generally can be performed for an essentially unlimited time. This also allows carrying out time-lapse assays, as well as dynamical characterization of the heterogeneity of single cell behavior. Hence, the experimental results that can be obtained by the apparatus of the invention on single tumor cells can be statistically analyzed in order to determine the heterogeneity based on analysis carried out with single tumor cells.

In addition to stimulating cell heterogeneity by confining a single cell phenotype, cell heterogeneity can also be *in vitro* mimicked with the apparatus of the invention, for example, by co-immobilizing different tumor cell phenotypes within the same microcapsules (i.e. MCF7 and MCF10A), or immobilizing the same tumor cell type once pre-cultured in the presence of an activator or growth factor (i.e. MCF10A in presence or absence of TGF-β) and once in the absence of an activator or growth factor. Also more complex cell populations comprising tumor cells may be entrapped, for example, cells from a tumor biopsy.

In summary, with the apparatus of the invention it is now possible to properly mimic the tumor tissue milieu in a 3D-1D configuration and to mimic both the mechanical and textural properties of the tumor tissue niche.

Furthermore, within the apparatus of the invention it is now also possible to perform *in vitro* single tumor cell assays derived from multicellular aggregates, cellular microspheroids, and/or multicellular populations immobilized/generated within/from environments. Thus, the apparatus of the invention *inter alia* offers the following advantages:
a) The apparatus represents a new kind of high-through-put screening platform for *in vitro* drug screening and/or studying and/or characterizing *in vitro* cells in the presence or absence of physical, biological or chemical stimuli. The apparatus is characterized by mimicking the mechanical (i.e. soft environments), topographical (3D, 1D) and biophysical stimuli (i.e. cell polarization induced by the pattern width) of tumor tissue *in vivo.*
b) The apparatus represents a new platform to determine cell heterogeneity at single-cell scale, based on the fragmentation of multicellular aggregates and/or microspheroids released from the microcapsules to the migration paths, which can just support the adhesion and spreading of single tumor cells.
c) The microcapsule allows discriminating between tumor cells, and healthy or non-tumor cells. When entrapping cell populations constituted by more than one cell type, the apparatus allows tumor cells to be separated from healthy or non-tumor cells. While tumor cells grow within and are released from the capsules, healthy or non-tumor cells are not able to do so, and die within the capsules.
d) The apparatus is a modulable and flexible system, capable to be arranged and performed with different types of microcapsules and/or migration paths.
e) The apparatus is able to support dynamic assays of tumor cell behavior, such as the existence of spatiotemporal events occurred during cancer progression. These assays can be carried out for an essentially unlimited time.
f) The apparatus may diminish the use of animals for cytotoxicity/pharmacological tests, as well as other biological/biophysical assays conducted to study the behavior of cells.
g) The apparatus can be used with cells isolated from human biopsies, including the existence of multiples cell populations (i.e. neoplastic or cancer-associated cells).
h) Due to the mechanical and biophysical properties characterizing the tumor-like microcapsules and the unique capability of cancer cells to proliferate and be released from confinement with respect to healthy cells and/or cancer-associated cells, the apparatus does not require that the population of neoplastic cells found in a human biopsy should be pre-isolated, pre-expanded, or increased in number prior to encapsulating them (*i.e*. using FACS), representing a clear advantage for saving time and resources when analyzing the pathogenicity of cancer cells, compared to current strategies used in the clinic.

To the best knowledge of the inventor there is currently no other apparatus or device having all these advantageous characteristics, thereby providing the biomedical industry with a new apparatus for studying the spatiotemporal behavior of single tumor cells and assaying tumor cell heterogeneity *in vitro.*

The present invention relates in a second aspect to a kit for analyzing single tumor cells comprising (i) a hydrogel-based polymeric microcapsule for entrapping cells, (ii) a hydrogel having on its surface a single cell migration path being formed by one or more extracellular matrix proteins and/or one or more polymers comprising RGD peptides or proteins, and (iii) optionally instructions how to use the kit.

The definitions and preferred embodiments of the first aspect of the invention apply *mutatis mutandis* to the second aspect of the invention. In this connection it is to be understood that the kit comprises the various components of the apparatus of the invention which are not yet assembled into the apparatus of the invention. Hence, the kit advantageously allows preparing an apparatus according to the operator's wishes and/or the experimental needs.

For example, also for the kit it is increasingly preferred that it comprises at least 2, at least 3, at least 4, at least 5, at least 10, at least 20, at least 50 or at least 100 microcapsules. It is also increasingly preferred that the kit comprises a number of migration paths, so that each microcapsule can be co-localized with, positioned or placed in direct contact with or connected to at least 2, at least 3, at least 4, at least 5, at least 10, at least 20, at least 50 or at least 100 migration paths.

The various components of the kit may be packaged into one or more containers such as one or more vials. The vials may, in addition to the components, comprise preservatives or buffers for storage. The kit may comprise instructions how to use the kit, which preferably inform how to use the kit for preparing an apparatus of the invention.

In accordance with a preferred embodiment of the first and second aspect of the invention, the hydrogel is immobilized on a solid carrier.

The incorporation of a solid carrier is advantageous since it provides the hydrogel with additional physical strength, so that the apparatus is less fragile and easier to handle. Due to the solid carrier the hydrogel may also be placed more easily onto a portable platform, such as a portable platform of a fluorescent and confocal microscope.

The term "immobilized" or "immobilizing" relates to the fixation of the hydrogel on the solid carrier. Said immobilization may be irreversible or reversible. Fixation may be due to covalent or non-covalent bonds formed between the material of the carrier and the hydrogel. Exemplary non-covalent interactions include those leading to adsorption. The fixation of the hydrogel to the solid carrier can be performed by any technique known in the state of the art (see, for example, Hermanson, G.T., Bioconjugate Technique (2008), 2nd edition):
The term "solid carrier" defines a carrier of solid material. The material of the carrier may be either of compact or porous structure. The carrier may be, for example, of a material selected from the group consisting of glass, plastics, medical grade stainless steel, metal alloys (e.g. chrome cobalt molybdenum, titan nitride oxide), hydroxyapatite, silicone, polystyrene, poly-L-lactic acid; polyurethane, polyester, polysulfone, polyethylene, polypropylene, polyacryl, polyacrylnitril, polyamid, PMMA (*para*-methoxy-*N*-methylamphetamine), fleece wadding, open porous foam plastic or glass and reticular plastic or glass and structures derived from marine sponges (porifera). The solid carrier is preferably of glass or is a cell culture plate or a vial, noting that a cell culture plate or a vial is usually made of plastics.

In accordance with a further preferred embodiment of the first and second aspect of the invention, the polymeric microcapsule is a degradable polymeric microcapsule, preferably wherein the degradable polymeric microcapsule becomes degraded (i) upon culturing cells being entrapped in the degradable polymeric microcapsule under growth conditions in a cell culture medium, (ii) upon contact with a chelating agent, or (iii) upon contact with an enzyme.

As regards feature (i) it to be understood that as a result of culturing the tumor cells in the microcapsule the microcapsule becomes degraded. This can be the result of physical strength of the growing tumor cells, so that the microcapsule becomes degraded and/or a process of biodegradation of the microcapsule in the culture medium. In the latter case the biodegradation of the capsule may be triggered by the secretion of enzymes or other cellular components from the entrapped tumor cells. Among features (I) to (III) feature (I) is preferred since this degradation mode is illustrated by the examples herein below.

While feature (i) does not require a step of adding a further compound into the apparatus, features (ii) and (iii) require the addition of a chelating agent or an enzyme into the system. A chelating agent is a substance whose molecules can form bonds to a single metal ion, thereby preventing the metal ion forming bonds to other compounds. As will be further detailed herein below, the polymer forming the microcapsule may be formed by polymers that are cross-linked by cations, in particular metal ions such as calcium ions. Hence, upon the addition of a chelating agent the cross-links are removed and the microcapsule becomes degraded. Examples of chelating agents are ethylenediaminetetraacetic acid (EDTA) and ethylene glycol-bis(β-aminoethyl ether)-N,N,N',N'-tetraacetic acid (EGTA). An enzyme is a protein that acts as a catalyst, regulating the rate at which chemical reactions proceed without itself being altered in the process. Various enzyme are known which are capable of degrading polymers and thus also the polymeric microcapsule (A. Banerjee, K. Chatterjee & G. Madras (2014) Enzymatic degradation of polymers: a brief review, Materials Science and Technology, 30:5, 567-573). For example, alginate can be degraded by alginate lyases (for review: Zhu and Yin (Bioengineered. 2015 May-Jun; 6(3): 125-131) and chitosan by exo-β-d-glucosaminidase. Silk can be degraded, for example, by α-chymotrypsin, collagenase IA or protease XIV. The serine protease plasmin degrades fibronectin. Finally, gelatin can be degraded, for example, by cellulase (i.e. produced by Aspergillus niger), Novozym 435, gelatinase, collagenase, trypsin, pepsin or lysozyme.

Notwithstanding to the above options it is also possible to simply break up the microcapsule at a desired time by external physical strength, e.g. by a needle.

In accordance with a further preferred embodiment of the first and second aspect of the invention, the hydrogel is a polyacrylamide hydrogel or a polyacrylamide-allylamine hydrogel.

Polyacrylamide is preferably cross-linked to a hydrogel by tetramethylethylendiamin and ammonium persulfate.

Polyacrylamide and allylamine are preferably present at a ratio of between about 500:1 v/v to about 500:3 v/v and/or the polyacrylamide is preferably cross-linked to a hydrogel by tetramethylethylendiamine and ammonium persulfate.

Such hydrogels are used in the examples herein below and are particularly advantageous. While polyacrylamide is preferably used when the capsules are co-localized without being chemically bound, the blend polyacrylamide and allylamine is preferably used when the capsules are chemically connected to the hydrogel. Concerning the polyacrylamide and allylamine, this mixture is advantageous because the polyacrylamide allows generating a hydrogel with defined mechanical properties (27) while allylamine can be used to generate chemical cross-links between migration paths and microcapsules.

In accordance with another preferred embodiment of the first and second aspect of the invention, the polymeric microcapsule comprises or consists of gelatin and alginate, or chitosan and gelatin, or silk and polyethylene glycol and gelatin, or alginate and fibronectin.

All these different polymers are used preferably in a ratio of about 1:1(:1) (w/v) and/or preferably at a concentration of between about 0.5% w/v and about 2.0% w/v for each polymer.

Microcapsules made of these polymers are particularly advantageous since all combinations comprise a polymer which becomes biodegraded during cell culture and a polymer which is inert to the cell culture conditions. Alignate, chitosan, polyethylene glycol and silk are inert while gelatin and fibronectin are biodegradable upon growing cells within a capsule comprising or consisting of gelatin and/or fibronectin. Gelatin and alginate are most preferred since they are used in the examples.

By using a combination of a biodegradable and an inert polymer the timing of the release of tumor cells from the microcapsule (typically after about 5 days) as well as the numbers of tumor cells which are released per time (i.e. the pore size) can be adjusted by adjusting the concentration of the polymer types.

In accordance with a preferred embodiment of the first and second aspect of the invention, the migration path is formed by collagen, fibronectin, laminin, elastin, RGD-modified silk, RGD-modified alginate, and/or RGD-functionalized polyacrylamide.

As discussed above, collagen, fibronectin, laminin, elastin are the most abundant ECM proteins. RGD-modified silk, RGD-modified alginate, and RGD-functionalized polyacrylamide are examples of polymers comprising RGD peptides or proteins.

In accordance with a further preferred embodiment of the first and second aspect of the invention, the polymeric microcapsule has a diameter of about 100 µm to about 1200 µm.

The term about means preferably ±20%, more preferably ±10% and most preferably ±5% of the respective values indicated herein.

A diameter of about 100 µm to about 1200 µm is particularly preferred since it offers enough space to entrap cells and exerts upon the growth of the tumor cells physical strength on the tumor cells within the microcapsule.

In accordance with a yet further preferred embodiment of the first and second aspect of the invention, the single cell migration path has a width of about 1 µm to about 10 µm and/or a length of at least 50 µm.

The width is particularly preferred since it matches the dimensions of most cell types including tumor cells, so that only single-cells can migrate down the patch. Also the length is particularly preferred since it is sufficiently long to analyze the single cells along the path.

In accordance with a still further preferred embodiment of the first and second aspect of the invention, the polymeric microcapsule comprises a polymer that has been cross-linked by cations.

Cations can be used to cross-link polymers, preferably alginate. By adjusting the concentration of the polymers and the cations the number of cross-links (i.e. bonds between polymer chains) being formed can be adjusted.

These cations are preferably bivalent cations and most preferably calcium ions. Calcium ions are preferably used to cross-link alginate. All cations are preferably used at a concentration of about 0.2M to about 1.0M.

In accordance with another preferred embodiment of the first and second aspect of the invention, the polymeric microcapsule and the single cell migration path are connected by covalent chemical bonds.

A covalent chemical bond is the interatomic linkage that results from the sharing of an electron pair between two atoms. The binding arises from the electrostatic attraction of their nuclei for the same electrons. A covalent bond forms when the bonded atoms have a lower total energy than that of widely separated atoms.

The covalent chemical bonds are preferably ester bonds and more preferably N-hydroxysuccinimide (NHS) ester bonds. NHS is commonly found in organic chemistry or biochemistry where it is used as an activating reagent for carboxylic acids. Activated acids can react with amines to form amides, for example. It is particularly preferred that the connection is made by EDC (1-Ethyl-3-(3-dimethylaminopropyl)-carbodiimide) and NHS (N-Hydroxysuccinimid) (25).

The present invention relates in a third aspect to a method for analyzing single tumor cells comprising (I) (a) covering the apparatus of the invention by a cell culture medium, (b) entrapping one or more tumor cells in the degradable polymeric microcapsule, (c) culturing the tumor cells under growth conditions until single tumor cells from the microcapsule migrate down the single cell migration path, and (d) analyzing the single tumor cells on the migration path; or (II) (a) covering a hydrogel-based polymeric microcapsule by a cell culture medium, (b) entrapping one or more tumor cells in the polymeric microcapsule, (c) culturing the tumor cells under growth conditions until the release of single tumor cells from the microcapsule cells, (d) placing at least one released tumor cell of step (c) on a single cell migration path, so that the cells migrate down the single cell migration path, wherein the single cell migration path is on the surface of a hydrogel and is formed by one or more extracellular matrix proteins and/or one or more polymers comprising RGD peptides or proteins, and (e) analyzing the single tumor cells on the migration path.

The definitions and preferred embodiments of the first and second aspect of the invention apply *mutatis mutandis* to the third aspect of the invention and *vice versa.* For instance, also in step (c) of the method the tumor cells (as single cells or as spheroids) are released from the microcapsule as described herein above.

The method as defined in item (I) of the third aspect of the invention is directed to analyzing single tumor cells by the apparatus of the invention. Accordingly the present invention also relates to the use of the apparatus of the invention for analyzing single tumor cells. The method as defined in item (II) of the third aspect of the invention is directed to analyzing single tumor cells by first culturing the tumor cells under growth conditions in the microcapsule until their release and placing a tumor cell (i.e. by hand or an automated device) or several tumor cells one after another on migration paths on a hydrogel to study the migration of the cells. Hence, the method as defined in item (II) of the third aspect of the invention uses the two main components - the microcapsules and the paths - of the apparatus of the invention one after another.

The following relates to all aspects of the present invention.

The cells referred to herein are preferably mammalian tumor cells and more preferably human tumor cells.

Typical human tumor cells include, for example, Hela, MCF-7, MDA-MB-231, PC-3, SK-OV-3, A2780, OVCAR-3, CAOV3, and IGROV1.

The tumor cells can also be primary cells, such as cancerous cells isolated from a biopsy, cancer and/or cells isolated from a tissue, cancer cells isolated from blood fluids, cancer and/or cells isolated from lymph fluids. All these tumor cells are generally found *in vivo* in admixture with non-tumor or healthy cells.

Appropriate culture media and conditions for tumor cells and cell populations comprising tumor cells (e.g. biopsy samples) are known in the art. For instance and depending on the cells and their specific requirements, cell culture can be carried out in RPMI, Williams' E or DMEM medium optionally containing 10% (v/v) FCS, 2 mM L-glutamine, and/or 100 U/ml penicillin and/or streptomycin.

In accordance with a preferred embodiment of the third aspect of the invention, the method further comprises the step of contacting the tumor cells in the polymeric microcapsule and/or on the single cell migration path with (i) a test compound, and/or (ii) a physical stimulus.

A test compound is any compound which is known to have or is to be tested to have an effect on cells. Likewise a physical stimulus is any stimulus other than a compound which is known to have or is to be tested to have an effect on cells.

The test compound is preferably selected from a drug, a drug candidate, a growth factor, a growth inhibitor, an antibody, a cytokine, a nano- or micro-particle, a virus, a genetic marker, a genetic transfection reagent, and an intracellular or membrane marker or dye.

The physical stimulus is preferably selected from a laser, magnetic waves, mechanical waves, X-rays, gamma radiation, micro-injection, and nano-indentation forces using atomic force microscopy.

The test compound or physical stimulus may be contacted with the cells in the microcapsule, for example, to exert or investigate an effect on the cell growth. The test compound or physical stimulus may also be contacted with the cells in the path, for example, to exert or investigate an effect on the cell migration speed, morphology or forces exerted along the path.

In accordance with a further preferred embodiment of the third aspect of the invention, step (d) comprises analyzing the single tumor cells with respect to one or more of traction forces, indentation forces, migration speed, cell morphology, sensitivity to a drug, growth factor or growth inhibitor, expression of genes and/or proteins, mechanical properties of single adherent cells, a physiological modification or a genetic modification.

As discussed herein above, the apparatus, kit and process of the invention allow investigating several properties of tumor cells which are of interest for researchers including the development of new compounds and strategies to treat diseases, in particular cancer. The determination of one or more of traction forces, indentation forces, migration speed, cell morphology, sensitivity to a drug, growth factor or growth inhibitor, expression of genes and/or proteins, mechanical properties of single adherent cells, a physiological modification or a genetic modification of single cells are non-limiting but preferred results that can be obtained by the apparatus, kit and process of the invention and which results provide valuable information for finally developing new compounds and strategies to treat tumors, in particular cancer.

In accordance with a preferred embodiment of all three aspects of the invention, the tumor cells are comprised in a mixture of tumor cells and healthy cells associated with a tumor.

Healthy cells or cancer-associated cells may serve as a control as compared to the tumor cells. Healthy cells associated with a tumor are from the same tissue as the tumor cells but are normal cells and not tumor cells. Preferably, the tumor cells and/or healthy cells associated with a tumor were obtained from the same subject and more preferably are from the same biopsy.

Unless otherwise defined, all technical and scientific terms used herein have the same meaning as commonly understood by one of ordinary skill in the art to which this invention belongs. In case of conflict, the patent specification including definitions, will prevail.

All the sequences accessible through the Database Accession Numbers cited herein are within the scope of the present invention and also include potential future updates in the database, in order to account for future corrections and modifications in the entries of the respective databases, which might occur due to the continuing progress of science.

All amino acid sequences provided herein are presented starting with the most N-terminal residue and ending with the most C-terminal residue (N→C), as customarily done in the art, and the one-letter or three-letter code abbreviations as used to identify amino acids throughout the present invention correspond to those commonly used for amino acids.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis mutandis* to all appended claims.

The Figures show.
**Figure 1****:** Illustration of an apparatus of the invention. The 3D-1D Multidimensional Cancer-on-a-Chip is constituted by 3D tumor-like microcapsules and 1D migration surfaces (also named as migration paths). Both scaffolds are chemically bound and linked to a silanized glass (22 X 22 mm). Within 3D tumor-like microcapsules cells are immobilized. After biodegradation of the polymeric matrix forming the 3D tumor-like microcapsules, and/or by exertion of mechanical forces, cells are released. After cells break free from entrapment, delivered cells attach, spread and migrate as single cells on the 1D migration paths. Due to the width of the thin migration guidelines, only single cells can adhere to the patterned surfaces. Under these conditions, cells can be chemically, biologically or physically characterized (as single cells), in order to study the phenomenon of cell heterogeneity.

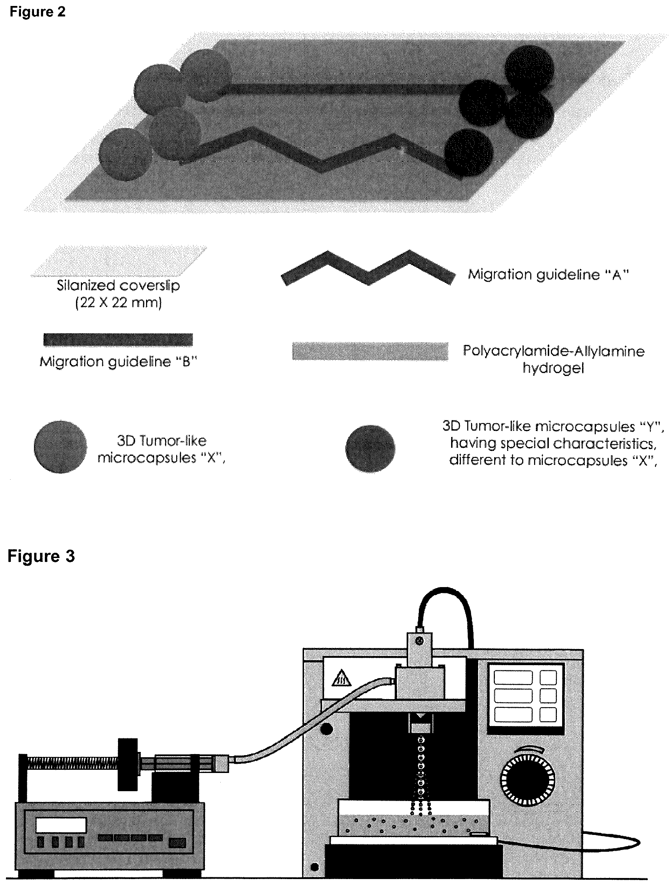

**Figure 2****:** Schematization of experimental options offered by the *3D-1D Multidimensional Cancer-on-a-Chip* described in this proposal. As shown, due to the modular design and performance, the *3D-1D Multidimensional Cancer-on-a-Chip* can be build with different cell types (or pre-cultured in different conditions), or microcapsules having defined mechanical properties, or having migration guidelines with distinctive features or fashions. In addition, the composition of microcapsules and patterned guidelines can be modified according to the operator wishes or the customer requirements
**Figure 3****:** Schematics of the encapsulation process and the encapsulator system used to perfom the 3D tumor-like microcapsules.

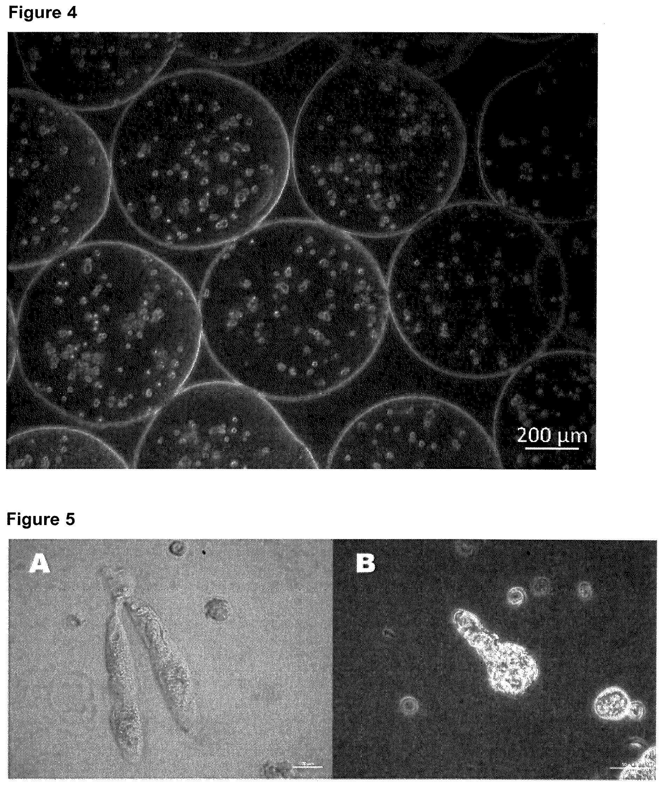

**Figure 4****:** Transmission light image of Tumor-like Microcapsules made according to the method previously described in this solicitude.
**Figure 5****:** Transmission light image of two different cellular spheroids (A, B) isolated from tumor-like microcapsules, according to the protocol described in this solicitude.

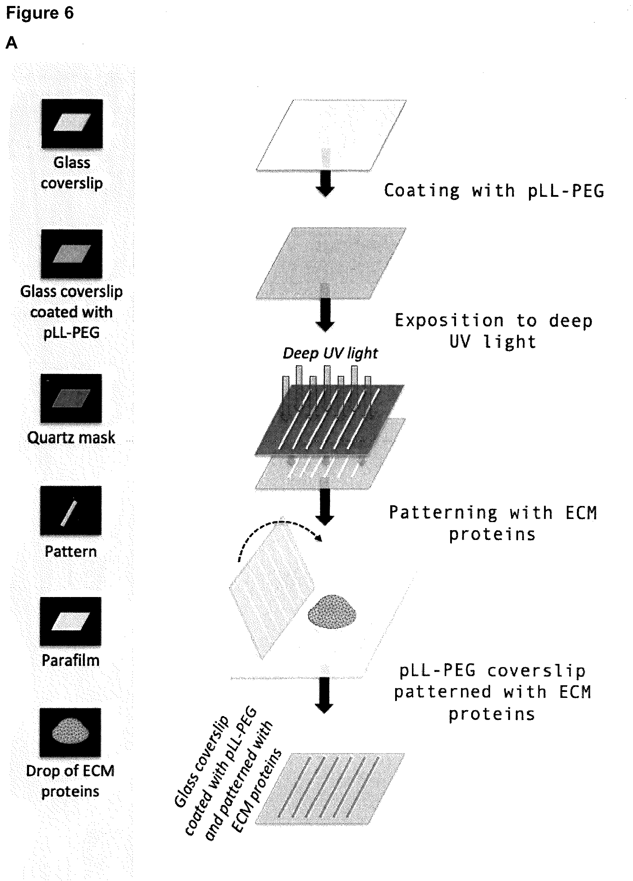

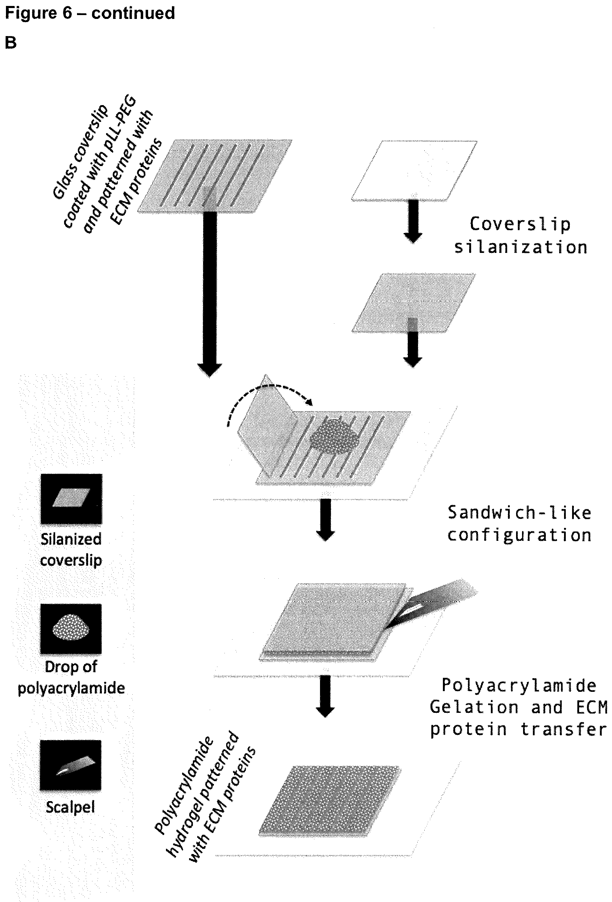

**Figure 6****:** (A) Schematization of the first protocol for preparing 1D micro-paths on Polyacrylamide hydrogels (first part); (B) Schematization of the first protocol for preparing 1D micro-paths on Polyacrylamide hydrogels (second part).

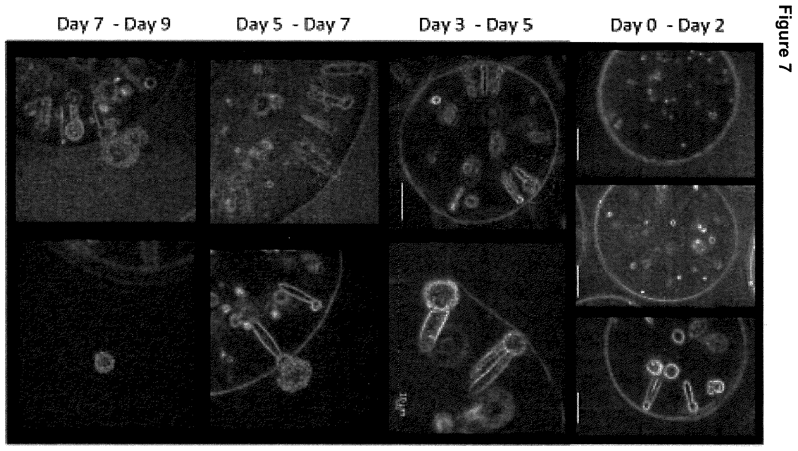

**Figure 7****:** Example of proliferation, aggregation, and release of MCF7 cells from tumor-like microcapsules. From day 0 to day 2, cell migration and early proliferation is observed. From day 3 to day 5 cells start growing producing forces (dotted circle), and degrading the wall of the tumor like microcapsules, to be released from confinement. From day 5 to 7 cells start being released from entrapment, mediated by the biomechanical and biophysical activity of cancer cells. From day 7 to day 9 it is possible to observe the existence of released spheroids or free cells, derived from entrapped cells in tumor-like microcapsules.

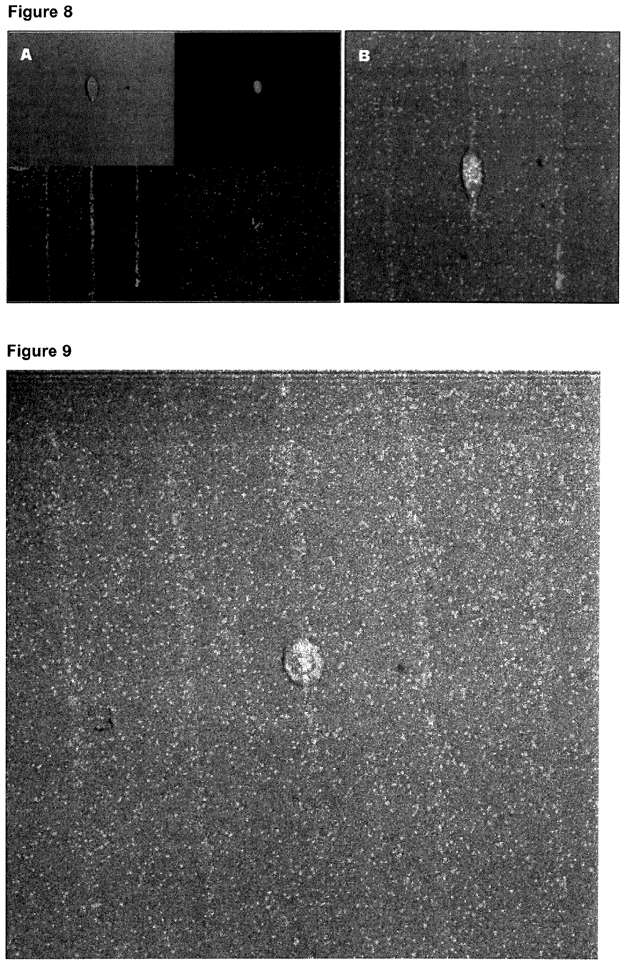

**Figure 8****:** Example of adhesion, polarization and migration of a single MCF7 cell delivered from a tumor-like microcapsules and anchored to one 1D path. Figure 8A shows single pictures of the different components of the system. On the top-left it is possible to see a single migrating cancer cell with reflected light. On the top-right the nuclei is detected, after staining with DAPI dye. On the bottom-left, the 1D paths of 2.5 µm width can be observed. These are made of Collagen type I. On the Bottom-right, it is possible to detect the presence of fluorescent microbeads, used to measure traction forces, as an example of biophysical characterization of the single neoplastic cell carried out with our system. Figure 8B shows the merge of all these images in one single picture.
**Figure 9****:** Adhesion and migration of a single cancer isolated from tumor-like microcapsules and anchored to 1D paths. In this image, red color shows the existence of 1D migration paths (2.5 µm width). The small dots represent shows the existence of fluorescent microbeads in the polyacrylamide hydrogel. The brilliant color in the center of the cell depicts the nucleus. The lines are the 1D paths, made of Collagen type-I.
**Figure 10****:** Determination of mechanical properties of tumor-like microcapsules. Image A shows the measurement of external elasticity (measured as Young's moduli) of tumor like microcapsules made of 1.0% or 1.5% w/v of alginate/gelatin blend, and crosslinked with different concentrations of CaCl₂ (from 0.2 to 1.0 Molar). Image A shows the measurement of external elasticity. Image B shows the measurement of the internal elasticity (after cutting the spheres in two half-spheres) in the same conditions descried previously.
**Figure 11****:** Comparison of cell-matrix adhesion forces (i.e. traction forces) measured *in vitro* (A), and cell extravasation determined *in vivo* (B), determined with MCF7 cells. Image A shows the exertion of traction forces by MCF7 cells pre-cultured on 2D cell culture flasks (left column) and MCF7 cells pre-cultured within tumor-like microcapsules (right column). Image A shows that MCF7 cells exerted higher traction forces when pre-cultured within tumor-like microcapsules with respect to those pre-cultured on 2D cell culture flasks. Image B shows results of MCF7 extravasation assays (i.e. number of infiltrated MCF7 cells in several zebrafish tissues, after their injection in the zebrafish bloodstream). Figure B shows that MCF7 cells pre-cultured in tumor-like microcapsules have a higher cell-tissue adhesion and invasive capability *in vivo* (left row), compared to same cell type pre-cultured on 2D cell culture flasks (right row).

### Example 1 - Material and Methods

### 1. Performance of Tumor-like microcapsules.

### 1.1 Preparation of the Alginate-Gelatin Blend.

Alginic acid sodium salt from brown algae was purchased from Sigma Aldrich (CAS: 9005-38-3). Gelatin type A was sourced from Sigma Aldrich from porcine skin (CAS: 9000-70-8).

The hydrogel blend material was prepared in the following manner: First, the desired amount of sodium alginate was dissolved in ultra-pure water (Milli-Q) under constant stirring. 1.0 % w/v of sodium chloride (J.T. Baker™, CAS: 7647-14-5) and 1.0 % w/v of HEPES buffer (Carl Roth®, CAS: 7365-45-9). When the alginate was completely solved, the gelatin powder was introduced to the solution.

After further stirring of the blend material, 10 N sodium hydroxide solution (EMPLURA®, CAS: 1310-73-2) was added until the pH-value of the solution reached a value between 7.3 and 7.6. The mixture was covered and heated to 45 °C and subsequently filtered under sterile conditions with a membrane filter (Carl Roth®, pore size 0.45 µm). Until encapsulation, the blend material was stored in a conical centrifuge tube from Falcon™.

All chemicals were used as received without any further purification.

### 1.2. Cell culture

Cells (i.e. MCF7, MCF10A, MDA-MB-231, EA.hy929) were cultured in T75 flasks (Sarstedt) with culture medium (GibcoTM, Cat.N°.: 11995065) until the culture reached a confluence of 80 - 90 %. For MCF7, MDA-MB-231 and EA.hy929, DMEM (Gibco Cat.N°.: 41966-029) was supplemented with 10 v/v % of fetal bovine serum (Corning, Cat. N° 35-079-CV) and 1.0 v/v % of penicillin-streptomycin (Corning, Cat.No.: 30-002-CI & MT35015CV).

In the case MCF-10A, these cells were cultured in DMEM/F12 nutrient mixture (GibcoTM, Cat.No.: 11330032) with the addition of 2.0 v/v % horse serum (GibcoTM, Cat.No.: 16050122), 1.0 v/v % penicillin-streptomycin (Corning, Cat.No.: 30-002-CI), 20.0 ng/ml epithelial growth factor (EGF, Sigma-Aldrich, CAS: 62253-63-8), 0.5 mg/ml hydrocortisone (Sigma-Aldrich, CAS: 50-23-7), 100 ng/ml cholera toxin (Sigma-Aldrich, CAS: 9012-63-9) and 10.0 µg/ml insulin (Sigma-Aldrich, CAS: 11070-73-8).

### 1.3. Encapsulation process

Cells were harvested until they reached a confluence between 80 and 90 %. When ready to use, cells were detached from the culture flasks with the help of 0.25 % Trypsin-EDTA (GibcoTM, Cat.N°.: 25200056). Cell counting was conducted with an improved Neubauer counting chamber. Next, cells were centrifuged with an Eppendorf centrifuge type 5804R at 1,200 rpm for five minutes and the supernatant was discarded, with the purpose to separate the sediment from the medium. The pre-tempered hydrogel blend (37 °C) was introduced to the cell pallet and mixed to achieve a homogenous cell-material mixture. The amount of material was carefully chosen to receive a cell concentration of approximately 500,000 cells/ml.

Further, the cell-material mixture was transferred into a syringe and installed in a syringe pump (World Precision Instruments, type AL-1000). A commercially available encapsulator (Büchi, B-390) was used to produce microbeads with the cell-blend mixture.

The nozzle size was defined according to the encapsulation requirements. In this example, a 300 µm nozzle size was chosen, with the purpose to obtain microbeads with a size of around 600-700 µm in diameter. Before encapsulation, the bead production unit (BPU), i.e. magnet, magnet holder, membrane, Luer lock etc., was preheated to 45 °C.

It should also be noted, that the entire encapsulation process was carried out under a sterile laminar flow cabinet, with the purpose to avoid contamination. Prior to the immobilization, the entire BPU was sterilized using an autoclave (Systec DX-23 or VX-65, 121 °C sterilization temperature). A sketch of the experimental construction can be seen Figure 3. The process parameters, which included flow rate, membrane frequency and electrode voltage, were experimentally redefined for each individual extrusion with cell-free material before the proper experiment was taken place.

Before starting the encapsulation process, the syringe containing the cell-material was kept sterile at 37 °C.

Then the material was extruded into a previously prepared and sterilized CaCl₂ solution (EMSURE®, CAS: 10035-04-8). This process is carried out under constant stirring where the gelling of the material took place.

The gelling bath was elevated in order to ensure better capsule homogeneity. The microbeads were left in the gelling bath for ten minutes after which they were rinsed with 0.1 M HEPES solution for another ten minutes. Following the drainage of the HEPES solution, beads were transferred into a six-well plate and stored in an incubator (37 °C, 5.0 % CO₂) with 3 ml of appropriate culture medium in each well. For the experiments, the used medium was changed once a week or earlier, if the cell medium displayed rapid change of color.

Figure 4 shows an image of the tumor-like microcapsules generated with the previously described system.

### 1.4. Dissolution of Tumor-like microcapsules.

When required, the capsules can be dissolved and cells released. This can be done using the following protocol:
After placing a whished amount of tumor-like microcapsules capsules in a 15 ml tube, these should be gently washed 2 times with 0.1 M Heppes buffer (pH 7.4), followed by a centrifugation step (300 rpm for 3 min).

As next, 1.0 ml of a solution containing Na-Citrate (22,5mM), EDTA (60mM), NaCl (150mM) (pH 7.4) was added to previous pellet. From this solution cellular aggregates can be obtained in solution, as Figure 5 shows.

The solution should be softly stirred for a maximum of 5 minutes. Further, the solution should be centrifuged at 1000 rpm for 5 minutes, followed by a trypsinization stage (0.5mL, 2 minutes) with the purpose to obtain single cells from aggregates found within the tumor-like microcapsules. Trypsin is stopped by using of cell culture media (3.5 mL).

After a final step of centrifugation, cells (5 min 1000 rpm), the cells are re-suspended in cell culture media, before to seed on surfaces having 1D paths.

### 2. Performance of 1D micropatterned surfaces.

### 2.1 Preparation of Polyacrylamide Hydrogels.

Polyacrylamide hydrogel was deposited onto 22x22 mm glass coverslips (Fisherbrand, Massachusetts, USA). Before the coverslips were used, they were cleaned of any impurities to ensure the best possible hydrogel adhesion. Three-step process of alternate ultrasonication of coverslips in 70% isopropanol and milliQ water was carried out. After each step, coverslips were dried thoroughly with compressed air.

After cleaning, the coverslips were either autoclaved or silanized. Silanized coverslips were stored at 4.0°C for up to four weeks.

The deposition of Polyacrylamide hydrogel onto a glass coverslip was done following published protocols (Vignaud et al. (2014) Methods Cell Biol. 120, 93-116). An overview of the procedure can be seen in Figure 6a and Figure 6b.

The first step in hydrogel substrate preparation was silanization of the 22x22 mm coverslips by treatment with Bind Silane ([3-(Methacryloyloxy)propyl]trimethoxysilane, PlusOne Bind-Silane; GE Healthcare Life Sciences). This procedure is required for covalently bind the gel to the glass.

As second step on the preparation of 1D micropatterned surfaces, clean 24X24 mm coverlisp were exposed to plasma (5 minutes) and coated with 0.1 mg/ml pLL-PEG (PLL20K-G35-PEG2K, JenKem Technology, USA) (Vignaud *et al.,* 2014). pLL-PEG was used to avoid adhesion/adsorption of proteins to the glass surfaces.

As next, 24X24 mm pLL-PEG coated coverslips were contacted with a Glass-Quarz Photomask (Toppan, France), having drawn transparent migration guidelines on this.

After 5 minutes of exposing the 24X24 mm pLL-PEG to deep UV light average intensity of 253.7 nm), 75 µl droplets of 40 µg/ml Coll-I solution (Discovery Labware Inc., Massachusetts, USA) was deposited and onto the 24x24 mm pLL-PEG glass coverslips. Due to the UV light degrade the pLL-PEG under the pattern, Coll-I would be solely attached to the places where the UV light might trespass the mask (*i.e.* patterned surfaces) (Figure 6a).

After 30 minutes in a humidification chamber, those pLL-PEG coverslips, already coated with Coll-I were gently lifted and washed.

In the next step, the Polyacrylamide solution was prepared according to the protocols of Tse et al. (2010) Current Protocols in Cell Biology. 10.16.1-10.16.16. Fluorescent microbeads (FluoSperesTM carboxylate, 0.2 µm, crimson, Invitrogen) were also added to the acrylamide solution. The beads acted as fiducial markers for measuring cell traction forces (Figure 6b)
Once the beads were added, the solution was homogenized for 10 minutes by ultrasonic agitation to ensure complete homogeneity of the bead distribution.

Further, the crosslinker Ammonium-per-sulphate (APS, Cat. N°: A3678, Sigma Aldrich, USA) and the reaction accelerator TEMED (N,N,N0,N0-Tetramethylethylenediamine (Cat. N°: T9281, Sigma Aldrich, USA) were added to the acrylamide solution.

Droplets of 50 µl of the polymerizing solution were then quickly deposited onto the collagen-covered glass and covered with 22x22 mm silanized coverslip. The gel was left to polymerize between the two coverslips in this sandwich-like configuration for 25 minutes (Figure 5).

The gels were polymerized for 30 minutes. After that, a scalpel was used to detach the non-silanized glass from the glass containing the Polyacrylamide hydrogel anchored.

The thickness of the obtained hydrogel layer was approximately 100 µm. Once the polymerization was complete the substrates were kept submerged in milliQ water at room temperature (RT) and used for further experiment. *3. Performance of 3D-1D Cancer-on-a-Chip.*

The coverslips having the patterned Polyacrylamide on its surface was placed in a magnetic chamber (1 Well Chamlide CMS, for 22 X 22 mm slides; Live Cell Instrument, Seoul, Korea) and filled with 1 mL of cell culture media.

After that, several amount of tumor-like microcapsules were placed in the same media.

The culture of both scaffolds, placed in the same chamber, can be carried out for an unlimited amount of days.

### Example 2 - Study of cell proliferation within 3D tumor-like microcapsules, followed by their release and later adhesion to 1D micro-paths

Figure 7 depicts the progression of cell growth and further release from tumor-like microcapsules during 9 days.

As Figure 7 shows, cancer cells are able to migrate (day 0 to Day 2), proliferate and generate aggregates (within the red circle, day 3 to 5) and be released from the tumor-like microcapsules (day 7 to 9). Released cells are able to reach the patterned lines and start migrating on single guidelines.

It is important to note that under these conditions, all tested cancer cells (*i.e*. MCF7, MDA-MB-231) exhibited the same behavior. Otherwise, non-metastatic cell lines (*i.e*. MCF10A, EA.hy929) were able to survive, but not to proliferate or be released from entrapment, indicating that the culture conditions described herein are able to be used to discriminate between healthy from pathogenic cells.

After cell release from the tumor-like microcapsules, cells adhere, spread and migrate on 1D paths. Figure 8 shows a single released MCF7 cell attached, spread and migrating on a 1D path of 2.5 µm width.

As Figure 8 shows, cells are able acquire a polarized morphology on the 1D patterns, which is kept during cell migration.

### Example 3 - Study of cell proliferation within 3D tumor-like microcapsules, followed by the dissolution of the capsules and seeding of cells entrapped within 3D tumor-like microcapsules.

Tumor-like microcapsules were dissolved as described in a previous section of this report.

After cell isolation, cells were seeded on the surfaces having the 1D paths. Figure 9 shows an example of a single cell isolated from spheroids, and migrating on 1D paths.

### Example 4 - Study to test the in vivo reliability of the tumor-like microcapsules as a mean to resemble the tumoral architecture

When comparing the exertion of traction forces (i.e. adhesion forces) of cells pre-cultured on cell culture flasks (2D) with respect to those pre-cultured in the tumor-like microcapsules of the present invention for 5 days (3D), it is observed that entrapped cells have a strong cell-matrix adhesion to the cells cultured on 2D, as Figure 11 shows.

With the purpose to test the *in vivo* reliability of the tumor-like microcapsules as a mean to resemble the tumoral architecture, cells pre-cultured on culture flasks or those pre-cultured on tumor-like microcapsules for 5 days were injected in the bloodstream of zebrafish, and cell extravasation assays were carried out (Follain G et al., Dev Cell. 2018, 45: 33-52). Extravasation is known as one of the crucial steps in caner metastasis, refereed to the infiltration of neoplastic cells in a tissue different to the tumor, with the purpose to create secondary tumor. According to the experimental results, cancer cells pre-cultured on 3D exhibit higher extravasation rates compared to those pre-cultured on 2D, demonstrating the reliability of the tumor-like microcapsules as artificial milieu to resemble the biological and mechanical stimulation required for cancer cells to show their pathogenic capability.

### References

1. Bonnans C. et al. (2014). Remodelling the extracellular matrix in development and disease. Nat. Rev. Mol. Cell. Biol. 15, 786-801 .
2. Butcher D., et al., (2009). A tense situation: forcing tumour progression. Nature Reviews Cancer, 9: 108- 122.
3. Paul, C.D. et al. (2017). Cancer cell motility: lessons from migration in confined spaces. Nat. Rev. Cancer. 17, 131-140.
4. Jain, R.K. et al. (2014). The role of mechanical forces in tumor growth and therapy. Annu. Rev. Biomed. Eng. 16, 321-346.
5. Wirtz, D. et al. (2011). The physics of cancer: the role of physical interactions. Nat. Rev. Cancer. 11 ,512-522.
6. Lu, P. et al. (2012). The extracellular matrix: A dynamic niche in cancer progression. Journal of Cell Biology. 196, 395-406.
7. Doyle, A.O. et al. (2009). One-dimensional topography underlies three-dimensional fibrillar cell migration J. Cell Biol. 184: 481-490.
8. Doyle, A.O. et al. (2013). Dimensions in cell migration. Curr. Opin. Cell Biol. 25: 642-649.
9. Fisher R., et al. (2013). Cancer heterogeneity: implications for targeted therapeutics.
10. Meacham, C.E. and Morrison, S.J . (2013). Tumour heterogeneity and cancer cell plasticity. Nature. 501 : 328-337.
11. Alizadeh, A.A. et al. (2015). Toward understanding and exploiting tumor heterogeneity. Nature Medicine. 21 : 846-853.
12. Bedard P. et al. (2013). Tumour heterogeneity in the clinic. Nature. 501: 355-364.
13. Buczak, K. et al. (2018). Spatial tissue proteomics quantifies inter- and intra-tumor heterogeneity in hepatocellular carcinoma. Molecular and Cellular Proteomics. In the press. doi: 10.1074/mcp.RA117.000189.
14. Burrell R., Swanton C., (2016). Tumour heterogeneity and the evolution of polyclonal drug resistance. Molecular Oncology. 8: 1095-1111.
15. Polyak, K. (2011). Heterogeneity in breast cancer. J. Clin. Invest. 121 : 3786-3788.
16. Chen Y-H, et al. (2016). Single Cell Proteolytic Assays to Investigate Cancer Clonal Heterogeneity and Cell Dynamics Using an Efficient Cell Loading Scheme. Scientific Reports. 6: 27154.
17. Mendes Levitin H., et al. (2018). Single-Cell Transcriptomic Analysis of Tumor Heterogeneity. Trends in Cancer. 4: 264-268.
18. Shukla V., et al. (2018). Lab-on-a-Chip Platforms for Biophysical Studies of Cancer with Single-Cell Resolution. Trends in Biotechnology. 36: 549-561.
19. Khoo B.L., et al. (2016). Single.cell profiling approaches to probing tumor heterogeneity. International Journal of Cancer. 139: 243-255.
20. Lekka, M. (2016). Discrimination Between Normal and Cancerous Cells Using AFM. BioNanoSci. 6, 65-80.
21 . Afrimzon, E. et al. (2016) Hydrogel microstructure live-cell array for multiplexed analyses of cancer stem cells, tumor heterogeneity and differential drug response at single-element resolution. Lab Chip. 16, 1047-1062.
22. Wong A. et al. (2017). Drug screening of cancer cell lines and human primary tumors using droplet microfluidics. Scientific reports. 7: 9109.
23. Lovitt C., et al. (2014). Advanced Cell Culture Techniques for Cancer Drug Discovery. Biology (Basel). 3: 345-367.
24. Mosaad E., et al. (2018). The Microwell-mesh: A high-throughput 30 prostate cancer spheroid and drugtesting platform. Scientific Reports. 8: 253.
25. Leal-Egana A., et al. (2017). The size-speed-force relationship governs migratory cell response to tumorigenic factors. Molecular Biology of the Cell. 28: 1612-1621.
26. Vignaud T .. et al. (2014). Polyacrylamide Hydrogel Micropatterning. Methods in Cell Biology. 120: 93-116.
27. Tse J.R., and Engler A.J. (2001). Preparation of hydrogel substrates with tunable mechanical properties. Hoboken, NJ: John Wiley & Sons, Inc. doi: 10.1002/0471143030.cb1016s47.
28. Gilkes, D.M. et al. (2014). Hypoxia and the extracellular matrix: drivers of tumour metastasis. Nat. Rev. Cancer. 14: 430-439.
29. Martin J., et al. (2016). Reengineering the Tumor Microenvironment to Alleviate Hypoxia and Overcome Cancer Heterogeneity. Cold Spring Harb Perspect Med. 6: a027094.
30. Broders-Bondon F., et al. (2018). Mechanotransduction in tumor progression: The dark side of the force .Journal of Cell Biology. DOI: 10.1083/jcb.201701039.

## Claims

1. An apparatus for analyzing single tumor cells, said apparatus comprising a hydrogel-based polymeric microcapsule for entrapping cells and a hydrogel having on its surface a single cell migration path being formed by one or more extracellular matrix proteins and/or one or more polymers comprising RGD peptides or proteins, wherein the polymeric microcapsule and the path are placed in the apparatus such that upon the release of cells from the polymeric microcapsule a single tumor cell can migrate from the polymeric microcapsule on the path.

2. A kit for analyzing single tumor cells comprising
(i) a hydrogel-based polymeric microcapsule for entrapping cells,
(ii) a hydrogel having on its surface a single cell migration path being formed by one or more extracellular matrix proteins and/or one or more polymers comprising RGD peptides or proteins, and
(iii) optionally instructions how to use the kit.

3. The apparatus of claim 1 or the kit of claims 2, wherein the hydrogel is immobilized on a solid carrier.

4. The apparatus or kit of any proceeding claim, wherein the polymeric microcapsule is a degradable polymeric microcapsule, preferably wherein the degradable polymeric microcapsule becomes degraded
(i) upon culturing cells being entrapped in the degradable polymeric microcapsule under growth conditions in a cell culture medium,
(ii) upon contact with a chelating agent, or
(iii) upon contact with an enzyme.

5. The apparatus or kit of any proceeding claim, wherein the hydrogel is a polyacrylamide hydrogel or a polyacrylamide-allylamine hydrogel.

6. The apparatus or kit of any proceeding claim, wherein the polymeric microcapsule comprises or consists of gelatin and alginate, or chitosan and gelatin, or silk and polyethylene glycol and gelatin, or alginate and fibronectin.

7. The apparatus or kit of any proceeding claim, wherein the migration path is formed by collagen, fibronectin, laminin and elastin, RGD-modified silk, RGD-modified alginate, and/or RGD-functionalized polyacrylamide.

8. The apparatus or kit of any proceeding claim, wherein the polymeric microcapsule has a diameter of about 100 µm to about 1200 µm.

9. The apparatus or kit of any proceeding claim, wherein the single cell migration path has a width of about 1 µm to about 10 µm and/or a length of at least 50 µm.

10. The apparatus or kit of any proceeding claim, wherein the polymeric microcapsule comprises a polymer that has been cross-linked by cations.

11. The apparatus or kit of any proceeding claim, wherein the polymeric microcapsule and the single cell migration path are connected by covalent chemical bonds.

12. A method for analyzing single tumor cells comprising
(I)
(a) covering the apparatus of any one of claims 1 to 11 by a cell culture medium,
(b) entrapping one or more tumor cells in the polymeric microcapsule,
(c) culturing the tumor cells under growth conditions until single tumor cells from the microcapsule migrate down the single cell migration path, and
(d) analyzing the single tumor cells on the migration path; or
(II)
(a) covering a hydrogel-based polymeric microcapsule by a cell culture medium;
(b) entrapping one or more tumor cells in the polymeric microcapsule,
(c) culturing the tumor cells under growth conditions until the release of single tumor cells from the microcapsule cells,
(d) placing at least one released tumor cell of step (c) on a single cell migration path, so that the cells migrate down the single cell migration path, wherein the single cell migration path is on the surface of a hydrogel and is formed by one or more extracellular matrix proteins and/or one or more polymers comprising RGD peptides or proteins, and
(e) analyzing the single tumor cells on the migration path.

13. The method of claims 12, further comprising the step of contacting the tumor cells in the polymeric microcapsule and/or on the single cell migration path with
(i) a test compound, and/or
(ii) a physical stimulus.

14. The method of claim 12 or 13, wherein step (d) comprises analyzing the single tumor cells with respect to one or more of traction forces, indentation forces, migration speed, cell morphology, sensitivity to a drug, growth factor or growth inhibitor, expression of genes and/or proteins, mechanical properties of single adherent cells, a physiological modification or a genetic modification.

15. The apparatus, kit or method of any proceeding claim, wherein the tumor cells are comprised in a mixture of tumor cells and healthy cells associated with a tumor.
